# EUROPEAN PATENT APPLICATION

(11) **EP 4 721 646 A1**
(43) Date of publication of application: **08.04.2026**
(21) Application number: 24815922.0
(22) Date of filing: 31.05.2024
(51) Int. Cl.: A61B 5/00, A61B 5/374, G16H 50/20

(54) **METHOD AND APPARATUS FOR PROVIDING ACTIVITY INFORMATION OF CEREBRAL GLYMPHATIC SYSTEM**

(30) Priority: 31.05.2023 KR 20230070436
(71) Applicant: Seoul National University Hospital, Seoul 03080 (KR)
(72) Inventor: YUN, Chang-Ho, Seongnam-si, Gyeonggi-do 13620 (KR); LEE, Woo-Jin, Seongnam-si, Gyeonggi-do 13620 (KR)
(74) Representative: Dentons Patent Solutions Rechtsanwaltsgesellschaft mbH
(86) International application number: PCT/KR2024/007510
(87) International publication number: WO 2024/248548

(57) **Abstract**

Disclosed in the present specification are a novel and effective method and apparatus for measuring the activity level of the cerebral glymphatic system and a method and apparatus for screening a glymphatic system activity index. In one aspect, the method and apparatus of the present invention enable measurement of the activity of the glymphatic system with high predictive value and accuracy, and the method and apparatus of the present invention enable the discovery of polysomnography, brainwaves, and near-infrared spectroscopy indices for determining the activity of the glymphatic system.

## Description

### Technical Field

This specification describes a novel and effective method and apparatus for providing the activity information of the cerebral glymphatic system.

### Background Art

Recently, it has been discovered that the central nervous system also has a pathway that removes metabolites from brain cells to maintain stable brain cell functions, which is referred to as a "glymphatic system". In the glymphatic system, cerebrospinal fluid (CSF) moves along the perivascular space and enters the brain parenchyma through aquaporin-4 (AQP-4) water channels expressed in astrocyte endfeet surrounding the cerebral vasculature to be mixed with interstitial fluid, thereby removing waste products through solute exchange.

The brain accounts for 20% of the body's metabolic activity, resulting in the production of substantial metabolic waste products. Therefore, to maintain and promote brain health, it is crucial to maintain the appropriate activity of the glymphatic system. In particular, the glymphatic system plays an important role in removing pathological proteins, such as amyloid beta and tau proteins from the brain parenchyma, which cause neurodegenerative diseases, such as Alzheimer's disease. Additionally, the activity of the glymphatic system is known to increase by more than 60% during sleep and to be suppressed during wakefulness. Therefore, if the function of the glymphatic system is chronically impaired or if the glymphatic activation during sleep is insufficient, metabolic waste products including amyloid beta and tau proteins may not be properly cleared, thereby increasing the risk of developing neurodegenerative diseases, such as Alzheimer's disease, Parkinson's disease, amyotrophic lateral sclerosis, and Huntington's disease.

Considering the important role of the glymphatic system in normal brain function and pathophysiological aspects, objective and quantitative measurement of the activity of the glymphatic system is necessary. An existing quantitative method for measuring the glymphatic system includes two-photon microscopy, a quantitative method used in animal experiments. However, because this is highly invasive, it could not be applied to the human body. As a method for measuring the activity of the glymphatic system in the human body, some studies have attempted to inject gadobutrol into the cerebrospinal fluid and repeatedly acquire brain magnetic resonance imaging (MRI), or intravenously inject gadobutrol and repeatedly perform MRI scans to assess the activity and pathways of the glymphatic system. However, these approaches have several limitations. Intrathecal injection of gadobutrol has not been approved by the FDA, and real-time continuous MRI measurements are extremely difficult. In addition, it is impossible to track dynamic changes during wake-sleep transitions and sleep stages, these measurements interfere with natural sleep, and it is impossible to measure functions of the glymphatic system in various pathological conditions involving disruption of the blood-brain barrier. Therefore, the present inventors have developed a method for accurately measuring the brain water content in real time and non-invasively, using near-infrared spectroscopy (NIRS), thereby quantitatively measuring and monitoring the activity of the glymphatic system.

Previously, the factor determining the activity of the glymphatic system was known to be the compliance of cerebral arterioles. The cerebrospinal fluid moves along the periarterial space by the compliance of cerebral arterioles and perfuses into the brain parenchyma. Therefore, as vascular aging progresses, the compliance of cerebral arteries decreases, thereby reducing the function of the glymphatic system. Accordingly, vascular risk factors, such as hypertension, smoking, atherosclerosis, heart failure, and renal failure, have been proposed as risk factors for glymphatic system dysfunction. These risk factors share a common mechanism of impairing the compliance of cerebral arterioles through chronic vascular remodeling. However, there is a limitation in that it is difficult to reverse this chronic mechanism by modifying the risk factors.

Humans spend an average of 8 hours per day sleeping, and sleep has a major impact on overall health and quality of life. According to global sleep surveys, 60% of the total population is affected by sleep disorders and sleep disturbances, with insomnia (27%), snoring (24%), and sleep apnea (9%) which are the most common types of sleep disorders. Qualitative and quantitative deterioration of sleep is known to increase the risk of major diseases, such as dementia, cancer, stroke, cardiovascular diseases, hypertension, and diabetes. In particular, sleep disorders are known to increase the risk of dementia. Insomnia increases the risk of dementia by 1.5 times, sleep apnea by 1.2 times, sleep pattern changes by 1.9 times, and sleep behavior disorders (such as REM sleep behavior disorder) by 5.5 times. These risk levels are comparable to, or even higher than, those of the major risk factors for dementia that have been previously identified.

Previous pathophysiological hypotheses for dementia associated with sleep disorders involve decreased physical activity, depression, reduced concentration, and increased amyloid beta and tau protein production due to neural hyperactivity, and neuroinflammation. However, more recently, the decreased activity of the glymphatic system due to sleep disorders has emerged as a potential major cause of dementia. Sleep activates protein clearance mechanisms through the glymphatic system. According to mouse studies, a large amount of cerebrospinal fluid flows into the brain parenchyma immediately after sleep onset, and approximately 70% of amyloid beta clearance occurs during the first 30 minutes of sleep. This represents an approximately twofold increase in the clearance rate of amyloid beta, compared to wakefulness, suggesting that sleep plays a key role in regulating protein clearance mechanisms through the glymphatic system.

### [Prior Art Documents]

### [Patent Literature]

(Patent Document 0001) Korean Patent Application No. 10-2022-0166047 (Application Date: December 1, 2022)

### Detailed Description of the Invention

### Technical Solution

### [Cross-Reference to Related Applications]

This application claims the benefit of and priority to Korean Patent Application No. 10-2023-0070436, filed on May 31, 2023, entitled "Method for Measuring Cerebral Activity of the Glymphatic System", and Korean Patent Application No. 10-2024-0071504, filed on May 31, 2024, entitled "Method and Apparatus for Providing Activity Information of the Cerebral Glymphatic System", the disclosures of which are incorporated herein by reference.

The object of the present invention is to provide a novel method and apparatus for providing information on the activity of the glymphatic system and indices related to its determinants.

### Summary of the Invention

To achieve the above object, in one aspect, the present invention provides a method for providing activity information and/or activity indices of the glymphatic system.

The method performed by a processor comprises: analyzing changes in one or more brainwave indices and/or one or more near-infrared spectroscopy (NIRS) indices associated with changes in brain water content in the subject's brain; and providing activity information or activity index information of the glymphatic system based on the analysis results.

In another aspect, the present invention provides an apparatus for measuring the activity of the glymphatic system, the apparatus comprising: a first data processing unit that receives and analyzes information regarding one or more of NIRS indices including very-low-frequency oscillation and/or low-frequency oscillation of the subject's brain while the subject under sedation, and brainwave indices including alpha [8 to 12 Hz] and/or beta [13 to 30 Hz] frequency bands of a brainwave spectrum; and a second data processing unit that receives brain water content information of the subject and analysis results from the first data processing unit and provides activity information of the glymphatic system.

In another aspect, the present invention provides a computer-readable recording medium having program instructions stored thereon, the program instructions being executable by a processor of a computer and, when executed by the processor, cause the processor to perform the aforementioned method.

### Advantageous Effects of the Invention

In one aspect, the activity of the glymphatic system may be measured with high predictability and accuracy through the method and apparatus of the present invention.

In another aspect, the method and apparatus of the present invention may non-invasively measure the activity of the glymphatic system.

In another aspect, the method and apparatus of the present invention may provide a physiological mechanism for regulating the activity of the glymphatic system.

In another aspect, the method and apparatus of the present invention may identify polysomnography, brainwave, and near-infrared spectroscopy (NIRS) indices for determining the activity of the glymphatic system.

In another aspect, the method and apparatus of the present invention may provide standard data values for determining a normal range of the activity of the glymphatic system, thereby enabling the provision of information regarding whether the activity is normal or abnormal.

In another aspect, based on the measured activity of the glymphatic system, information on health indices (which may include lifestyle correction factors), diagnosis and/or progression, pathophysiology (including pathogenesis and etiology), and/or therapeutic efficacy of neurodegenerative brain diseases such as normal aging, Alzheimer's disease, Parkinson's disease, and/or other neurological disorders may be provided.

### Brief Description of Drawings

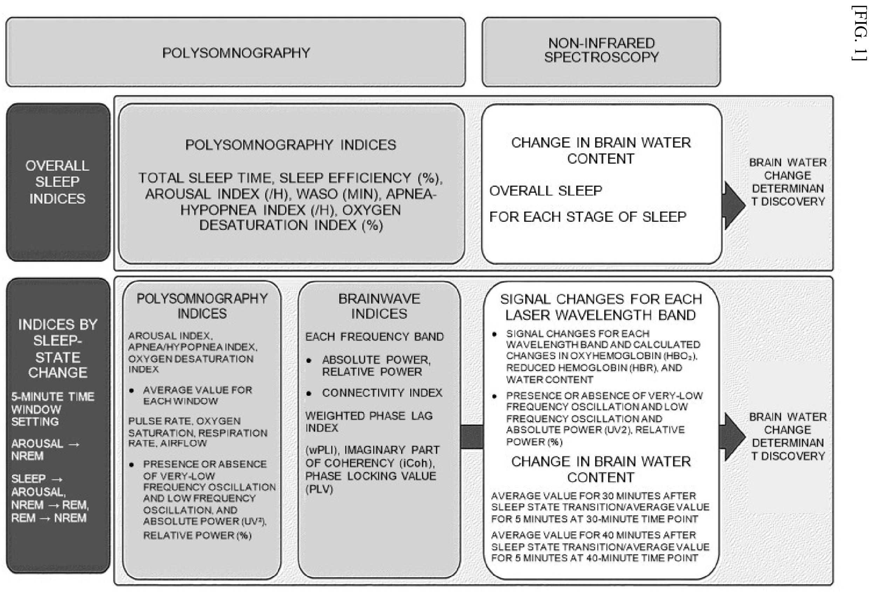

FIG. 1 is an overall schematic diagram of an experiment. In the experiment, the effect of overall sleep indices obtained from polysomnography on the total amount of change in brain water measured by near-infrared spectroscopy (NIRS) during sleep and the amount of change in brain water at each stage of sleep is analyzed. Specifically, to identify changes in brain water content at each stage of sleep and sleep-related factors affecting the same, a 5-minute time window is set, and various polysomnography indices, electroencephalogram indices, and NIRS indices are obtained for each time window. Subsequently, at 30-minute and 40-minute marks after a change in sleep state, the average change in brain water content during a 5-minute period is calculated and the effect of the indices on the average value of change in brain water content is analyzed.

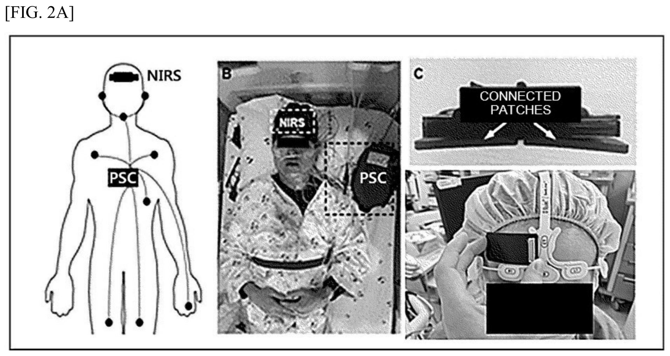

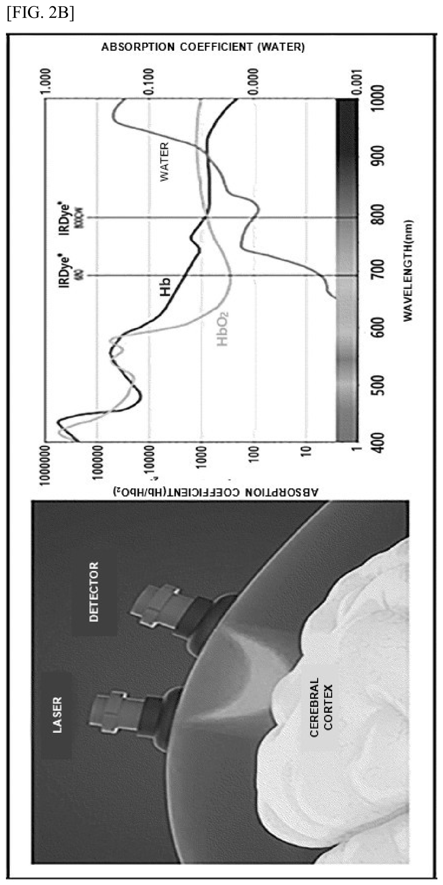

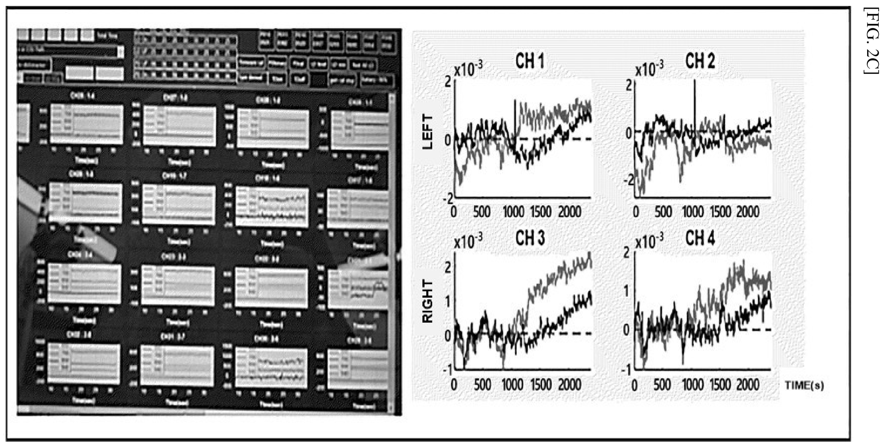

FIGS. 2A to 2C are diagrams illustrating a method for measuring changes in brain water content for each sleep stage based on NIRS. FIG. 2A relates to a method for measuring changes in brain water content for each sleep stage based on both NIRS and polysomnography (PSG). FIG. 2B is an overview of NIRS, wherein each laser wavelength band includes wavelength bands that exhibit specific absorbance for oxyhemoglobin (HbO₂), reduced hemoglobin (HbR), and water, respectively. Through this, the amounts of HbO₂, HbR, and water in the brain parenchyma may be calculated. FIG. 2C relates to a method for measuring brain water based on NIRS through multiple channels.

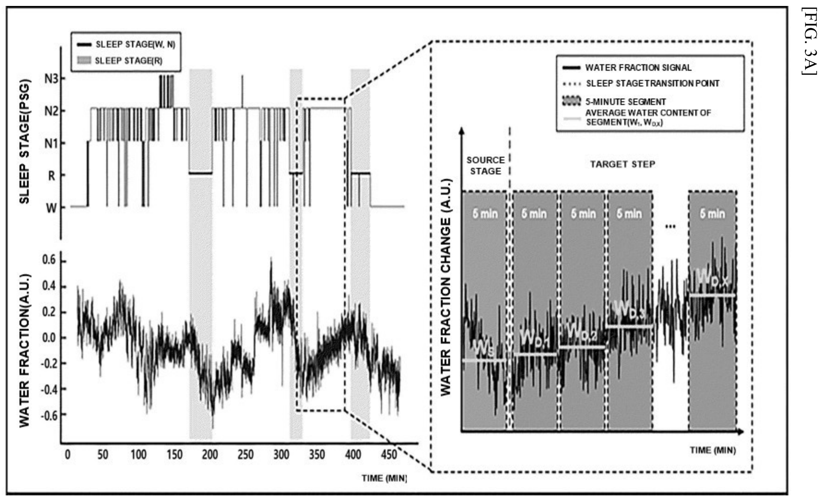

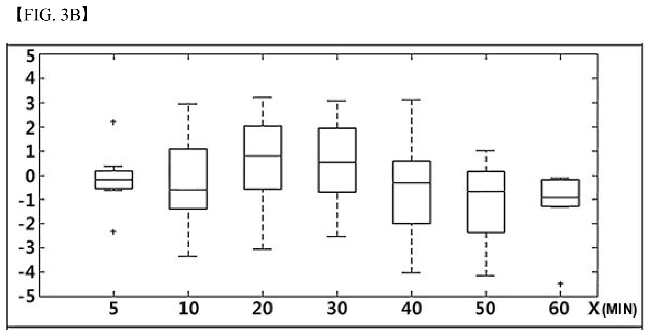

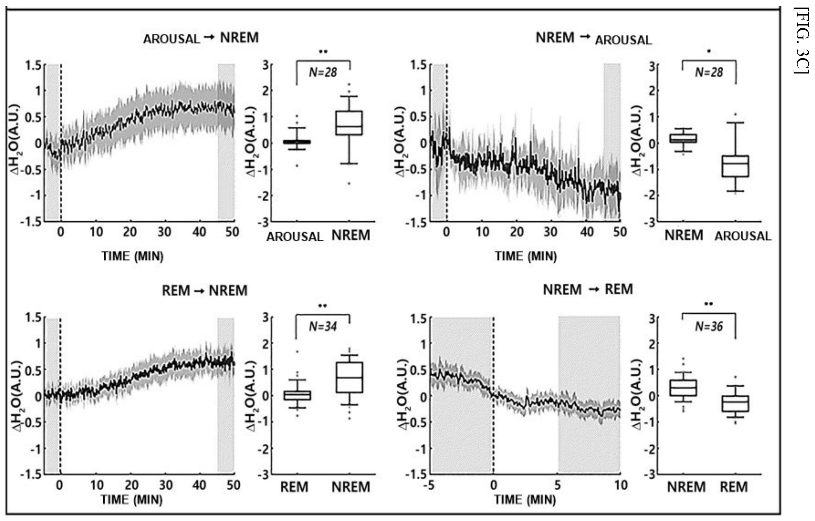

FIGS. 3A to 3C are diagrams illustrating a method for analyzing changes in brain water content for each sleep stage and changes in brain water content for each 5-minute time window. FIG. 3A illustrates measurements of changes in brain water content for each 5-minute time window matched to changes brain water content for each sleep stage. FIG. 3B illustrates a time-series comparison of changes in brain water content for each 5-minute time window over 60 minutes after a change in each sleep stage. FIG. 3C illustrates measurements of changes in brain water content for 5 minutes immediately before a change in each sleep stage and for 5 minutes at a specific time point after the change (45 minutes in this drawing).

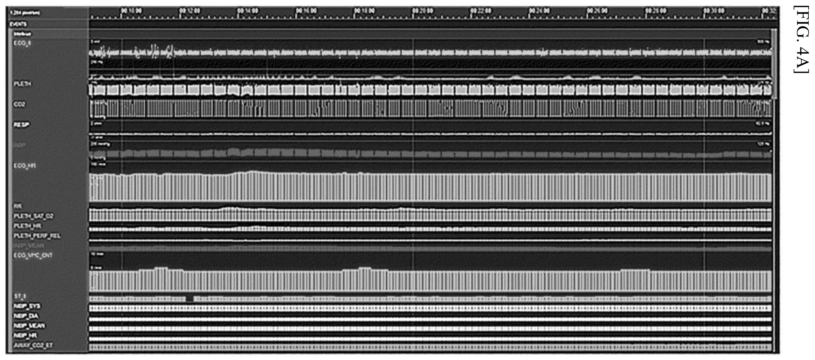

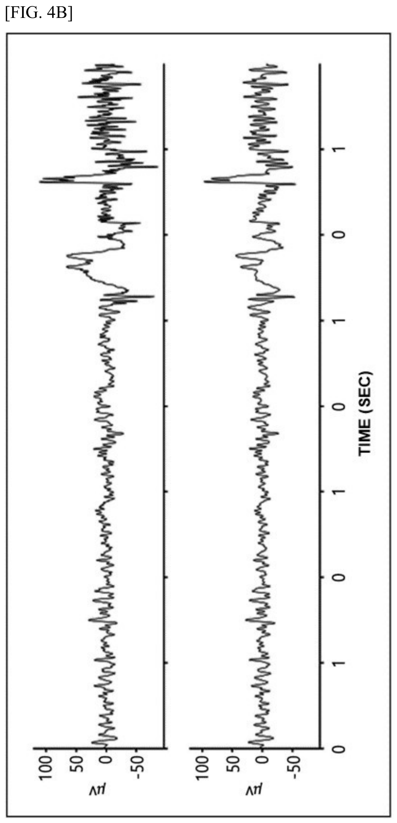

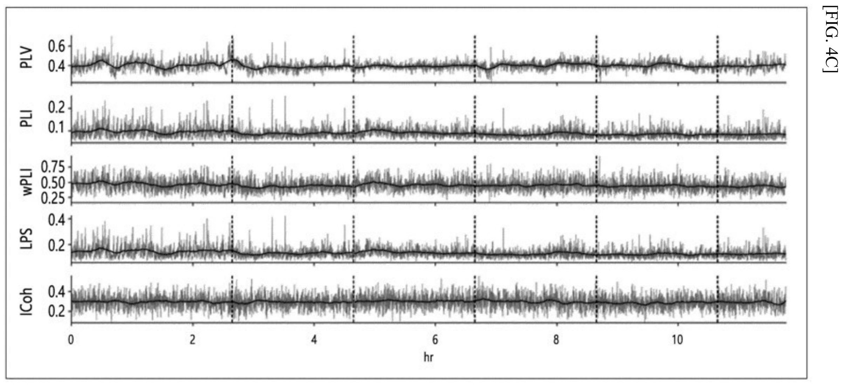

FIGS. 4A to 4C are diagrams illustrating a method for acquiring and analyzing brainwave indices and polysomnography indices. FIG. 4A illustrates a process of acquiring data and waveforms of brainwave and various sleep indices during polysomnography. FIG. 4B illustrates real-time acquisition of brainwave waveforms and amplitude data for each brainwave channel. FIG. 4C illustrates connectivity analysis performed based on the brainwave data.

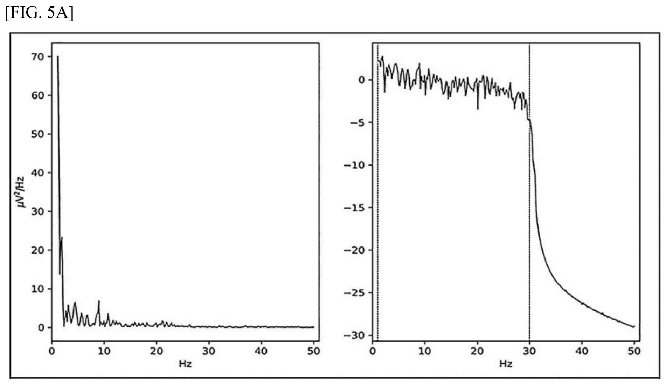

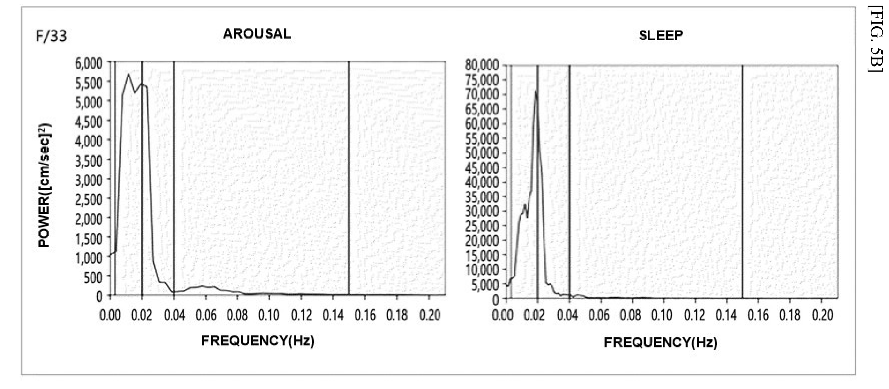

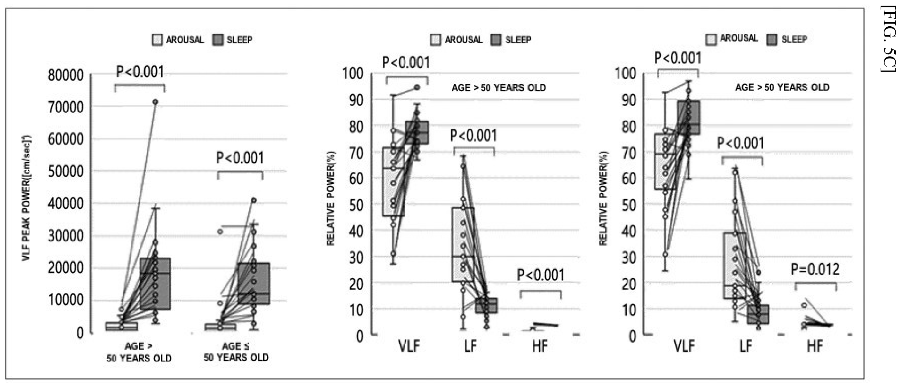

FIG. 5A illustrates power spectrum analysis. FIGS. 5B and 5C illustrate a method for verifying and quantitatively evaluating very-low-frequency oscillation and low-frequency oscillation. Specifically, FIG. 5B illustrates verification of the occurrence of very-low-frequency oscillation (at a high peak of 0.02Hz in this analysis) during sleep through power spectrum analysis based on real-time waveform data, such as hemodynamic indices including blood flow. FIG. 5C illustrates quantitative analysis of the absolute power and relative power of the very-low-frequency oscillation and low-frequency oscillation and comparative analysis of the power changes in the very-low-frequency oscillation and low-frequency oscillation according to each factor (age and sleep state in the drawing).

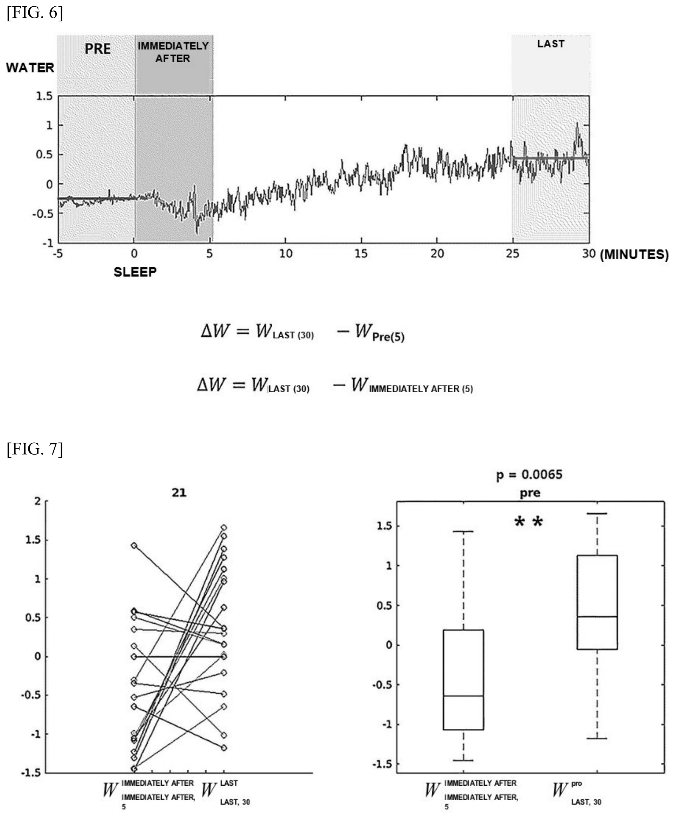

FIG. 6 illustrates analysis of changes in mean brain water content between the first 5 minutes immediately after sedative administration and the last 5 minutes at the 30-minute mark thereafter.
FIG. 7 illustrates the analysis results confirming that a brain water content increased at the 30-minute mark after administration of the sedative, i.e., dexmedetomidine, compared to the baseline.

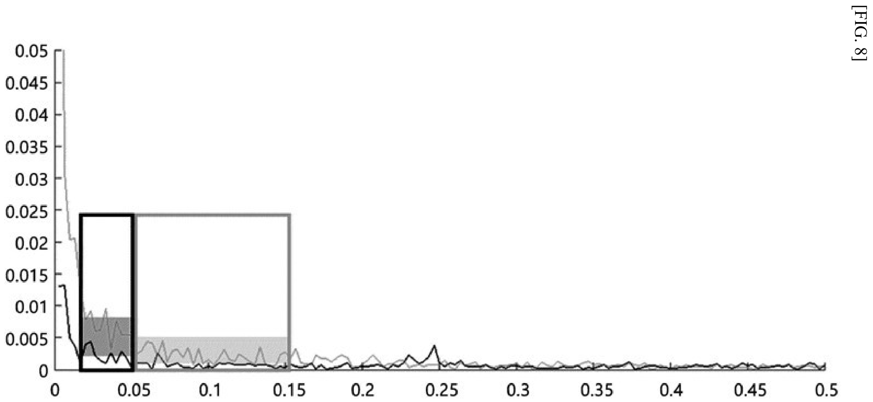

FIG. 8 illustrates an implementation example of power spectrum analysis for evaluating the power of frequency bands of very-low-frequency oscillation and low-frequency oscillation by Fourier transforming raw time series data of changes in brain water content.

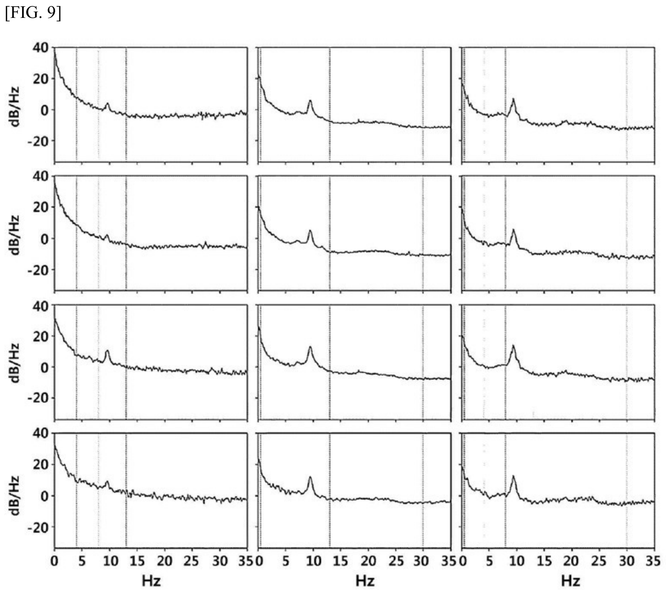

FIG. 9 illustrates an implementation example of power spectrum analysis for evaluating the power of each frequency band by transforming raw time-series data of brainwaves.

### Best Mode for Carrying Out the Invention

Exemplary embodiments of the present invention are described in detail below.

### Definition of Terms

In this specification, "activity information of the glymphatic system" includes activity information or activity indices of the glymphatic system.

In this specification, the activity of the glymphatic system may refer to the quantitative and/or qualitative activity level of the glymphatic system.

In this specification, the activity indices of the glymphatic system, which refer to markers associated with the activity of the glymphatic system may include one or more of various sleep indices including polysomnography indices, brainwave indices, and brain near infrared spectroscopy (NIRS) indices.

In this specification, terms such as "unit," "module," "apparatus," "terminal," "server," or "system" are intended to refer to a combination of hardware and software driven by hardware. For example, hardware may be a data processing device including a central processing unit (CPU) or other processors, such as a mobile device or a personal computer (PC). In addition, software driven by hardware may refer to a running process, an object, an executable, a thread of execution, a program, or the like.

### Description of Exemplary Embodiments

In one aspect, the present invention relates to a method for providing activity information or activity index information of the glymphatic system, performed by a processor, the method comprising: analyzing changes in one or more brainwave indices and/or one or more near-infrared spectroscopy (NIRS) indices associated with changes in brain water content in the subject's brain; and providing the activity or activity index information of the glymphatic system based on the analysis results.

In an exemplary embodiment, the analyzing step may include analyzing changes in polysomnography indices associated with changes in brain water content.

In an exemplary embodiment, the sleep indices obtained from polysomnography may be one or more selected from the group consisting of a total sleep time (minutes), a sleep efficiency (%), an arousal index (/h), a wake after sleep onset (min), an apnea-hypopnea index (/h), an oxygen desaturation index (%), a pulse rate, a respiration rate, and an oxygen saturation.

In an exemplary embodiment, the method may include: analyzing very-low-frequency oscillation and/or low-frequency oscillation of the subject's brain; providing brain water content information based on the analysis; and providing the activity information of the glymphatic system based on the brain water content information.

In an exemplary embodiment, the subject may be a subject whose activity of the glymphatic system has been increased by administration of a sedative, such as dexmedetomidine described below.

In an exemplary embodiment, the frequency of the very-low-frequency oscillation is from 0.0067 Hz to less than 0.04 Hz, and the frequency of the low-frequency oscillation is from 0.04 Hz to 0.1 Hz.

In an exemplary embodiment, the level of increase in relative power of the very-low-frequency oscillation and/or low-frequency oscillation has a correlation with the level of increase in brain water content and/or activity of the glymphatic system. This indicates that the level of increase in relative power of the very-low-frequency oscillation and/or low-frequency oscillation is a factor, i.e., a biomarker, that measures the physiological mechanism of an increase in brain water content and/or activity of the glymphatic system. Therefore, the level of increase in brain water content and/or activity of the glymphatic system may be predicted from the level of increase in relative power of the very-low-frequency oscillation and/or low-frequency oscillation.

In an exemplary embodiment, the analysis of changes in brainwave indices may include a power spectrum analysis of the brainwave. During the power spectrum analysis, absolute power (uV²) and/or relative power (%) of one or more frequency bands selected from the group consisting of delta [1 to 4 Hz], theta [4 to 7 Hz], alpha [8 to 12 Hz] and beta [13 to 30 Hz] may be measured.

In an exemplary embodiment, during the power spectrum analysis of the brainwave, absolute power (uV²) of alpha [8 to 12 Hz] and/or beta [13 to 30 Hz] frequency bands may be measured.

In an exemplary embodiment, the level of decrease in absolute power in the alpha and/or beta frequency bands is correlated with the level of increase in brain water content and/or activity of the glymphatic system. This indicates that the decrease in absolute power in the alpha and/or beta frequency bands is a factor, i.e., a biomarker, that measures the physiological mechanism of an increase in brain water content and/or activity of the glymphatic system. Therefore, the level of increase in brain water content and/or activity of the glymphatic system may be predicted by the level of decrease in absolute power in the alpha and/or beta frequency bands.

In an exemplary embodiment, the method and/or the apparatus may analyze connectivity within two or more frequency bands selected from the group consisting of delta [1 to 4 Hz], theta [4 to 7 Hz], alpha [8 to 12 Hz], and beta [13 to 30 Hz] of the brainwave spectrum.

In an exemplary embodiment, the method may further include analyzing connectivity within the alpha [8 to 12 Hz] and beta [13 to 30 Hz] frequency bands of the brainwave spectrum. The step of analyzing connectivity within the frequency bands may include measuring one or more indices selected from the group consisting of a weighted phase lag index (wPLI), an imaginary part of coherency (iCoh), and a phase locking value (PLV).

In an exemplary embodiment, the NIRS index may include presence or absence of very-low-frequency oscillation and low-frequency oscillation, and absolute power and relative power, in indices of water content as well as in each laser of the NIRS and in indices derived therefrom such as oxyhemoglobin (HbO₂) and reduced hemoglobin (HbR).

The wavelength of each laser of NIRS may be one or more selected from the group consisting of respective wavelengths that exhibit specific absorbance for HbO₂, HbR, and water. The amount of HbO₂, HbR, and water in brain parenchyma may be calculated through analysis of wavelength signals from each laser of NIRS.

In exemplary embodiments of the present invention, there is provided an apparatus for measuring the activity of the glymphatic system comprising: a first data processing unit that receives and analyzes information on one or more of: NIRS indices including very-low-frequency oscillation and/or low-frequency oscillation of the subject' brain while a subject is administered with a sedative; and brainwave indices including alpha [8 to 12 Hz] and/or beta [13 to 30 Hz] frequency bands of the brainwave spectrum; and a second data processing unit that receives brain water content information of the subject and analysis results from the first data processing unit and provides activity information of the glymphatic system.

Furthermore, in exemplary embodiments of the present invention, there is provided a computer-readable recording medium having program instructions stored thereon and operable by the computer. When the program instructions are executed by a processor of the computer, the processor performs the aforementioned method.

The computer may be a computing device, such as a PC, a desktop computer, a laptop computer, a notebook computer, a smartphone, or the like and may be any device that can be integrated into such a computing device. The computer may be a device having one or more general-purpose and special-purpose processors, memory, storage space, and (either wireless or wired) networking components. The computer may execute an operating system, such as an operating system compatible with Microsoft Windows, Apple OS X or iOS, Linux distribution, or Google's Android OS. The computer program includes a set of instructions executable on the aforementioned computer, and the name thereof may vary depending on the form of the computer. For example, when the form of the computer is a smartphone, the computer program may be referred to as an app.

In exemplary embodiments, the computer-readable recording medium storing a program for implementing the aforementioned method may include all types of recording device for storing computer-readable data. Examples of the computer-readable recording medium include read-only memory (ROM), random-access memory (RAM), compact disc (CD)-ROM, magnetic tape, floppy disk, optical data storage device, and the like. The computer-readable recording medium may also be distributed over a computer system connected via a network to store and execute computer-readable code in a distributed manner. Furthermore, functional programs, codes, and code fragments for implementing the present invention may be easily understood by those skilled in the art to which the present embodiment belongs.

### Detailed Description of the Invention

Hereinafter, the configuration and effects of the present invention are described in more detail with respect to embodiments. However, the following embodiments are provided for illustrative purposes only to aid in understanding of the present invention, and the scope of the present invention is not limited thereby.

### [Embodiment]

### Polysomnography and near-infrared spectroscopy monitoring method

Polysomnography is performed simultaneously with near-infrared spectroscopy (NIRS) monitoring. Through polysomnography, overall indices are extracted including total sleep time (minutes), sleep efficiency (%), arousal index (/h), wake after sleep onset (min), apnea-hypopnea index (/h), and oxygen desaturation index (%).

Sleep stages (wake, N1, N2, N3, REM) are classified for each 30-second epoch. When the first sleep epochs (N1, N2, N3) occur after wakefulness and the sleep epochs are maintained continuously for more than 2 minutes, it is determined that a transition from wakefulness to sleep has occurred. Transitions from sleep to wakefulness, from NREM sleep to REM sleep, and from REM sleep to NREM sleep are determined according to the same criteria.

Time windows are set at 5-minute intervals based on the time point of the sleep stage transition. Based on the time point of each time window, "5 minutes before transition" and "50 minutes after transition" are divided into time windows at 5-minute intervals. For each time window, sleep indices {arousal index (/h), apnea-hypopnea index (/h), and oxygen desaturation index (%)} are extracted.

Additionally, changes in brain water content by NIRS are measured for each time window. This represents a relative value measured based on the average value for 5 minutes before the sleep stage transition. For each time window, absolute power (uV²) and relative power (%) are measured for each frequency band (delta [1 to 4 Hz], theta [4 to 7 Hz], alpha [8 to 12 Hz], beta [13 to 30 Hz]) based on spectrum analysis of the brainwave data obtained from polysomnography.

Connectivity analysis is performed within each frequency band for each time window. The connectivity analysis includes indices, such as weighted phase lag index (wPLI), imaginary part of coherency (iCoh), and phase locking value (PLV).

For each time window, spectrum analysis is performed on indices, such as pulse rate, oxygen saturation, respiration rate, and airflow. Through this analysis, the presence or absence of very-low-frequency oscillation (0.01 to 0.1 Hz) and low-frequency oscillation (0.1 to 0.5 Hz) on each index is determined, and absolute power and relative power are measured.

For each time window, spectrum analysis is performed on wavelength signals of each laser of the NIRS and the calculated changes in oxyhemoglobin (HbO₂), reduced hemoglobin (HbR), and water content. Through this analysis, the presence or absence of very-low-frequency oscillation (0.01 to 0.1 Hz) and low-frequency oscillation (0.1 to 0.5 Hz) on each index is determined, and absolute power and relative power are measured.

### Analysis of sleep indices, brainwave indices and near-infrared spectroscopy indices

The sleep indices, brainwave indices, and near-infrared spectroscopy (NIRS) indices analyzed for each 5-minute window may include: (1) an arousal index (/h), an apnea-hypopnea index (/h), an oxygen desaturation index (%); (2) absolute power (uV²) and relative power (%) for each frequency band (delta, theta, alpha, beta); (3) a connectivity index for each frequency band (delta, theta, alpha, beta), such as weighted phase lag index (wPLI), imaginary part of coherency (iCoh), and phase locking value (PLV); (4) absolute power (uV²) and relative power (%) for each frequency band of polysomnography indices including a pulse rate, an oxygen saturation, a respiration rate, and an airflow; (5) presence or absence of very-low-frequency oscillation and low-frequency oscillation in indices including a pulse rate, an oxygen saturation, a respiration rate, and an airflow, and absolute power and relative power; and (6) presence or absence of very-low-frequency oscillation and low-frequency oscillation in each laser of NIRS and in indices derived therefrom such as the oxyhemoglobin (HbO₂), reduced hemoglobin (HbR), and water content, and absolute power and relative power.

The correlation between the indices for each 5-minute window and the changes in brain water content by NIRS is evaluated.

During transitions from wakefulness to NREM sleep, from sleep to wakefulness, from NREM sleep to REM sleep, and from REM sleep to NREM sleep, the following changes in brain water are measured by comparing: the average value of the brain water content for 5 minutes before transition with the average value of the brain water content for 30 minutes after transition; the average value of the brain water content for 5 minutes at the 30-minute mark; the average value of the brain water content for 40 minutes after transition, and the brain water content for 5 minutes at the 30-minute mark.

The correlation is analyzed between the brain water content indices, such as the average value of the brain water content for 30 minutes after the sleep state transition and the change index of the brain water content for 5 minutes at the 30-minute mark, and the sleep indices and brainwave indices, including the average value for 30 minutes and the window value for 5 minutes at the 30-minute mark.

The correlation is analyzed between the brain water content indices, such as the average value of the brain water content for 40 minutes after the sleep state transition and the change index of the brain water content for 5 minutes at the 40-minute mark, and the sleep indices and brainwave indices, including the average value for 40 minutes and the window value for 5 minutes at the 40-minute mark.

Through this, physiological mechanisms that regulate the activity of the glymphatic system may be discovered, and brainwave and polysomnography indices that determine the activity of the glymphatic system may be discovered.

### Analysis of brainwave indices and near-infrared spectroscopy indices according to drug use

Electroencephalography indices and near-infrared spectroscopy (NIRS) indices analyzed for each 5-minute window include: (1) absolute power (uV²) and relative power (%) for each frequency band (delta [1 to 4 Hz], theta [4 to 7 Hz], alpha [8 to 12 Hz], beta [13 to 30 Hz]) based on spectrum analysis of brainwave data; (2) presence or absence of very-low-frequency oscillation and low-frequency oscillation in brainwaves, and absolute power and relative power; (3) presence or absence of very-low-frequency oscillation and low-frequency oscillation in each laser of NIRS and in oxyhemoglobin (HbO₂), reduced hemoglobin (HbR), and water content indices derived therefrom, and absolute power and relative power.

The correlation between the indices for each 5-minute window and changes in brain water content measured by NIRS is evaluated.

While sedation is induced using a sedative, such as dexmedetomidine and/or propofol commonly used in operating rooms, the change in brain water content is measured by comparing the average value of the brain water content for 30 minutes after injection with the average value of the brain water content for 5 minutes at the 30-minute mark and the average value of the brain water content for 5 minutes immediately after sedative injection.

The correlation is analyzed between brain water content indices, such as the average value of the brain water content for 30 minutes after sedative injection and the change index of the brain water content for 5 minutes at the 30-minute mark, and the sleep indices and brainwave indices, including the average value for 30 minutes and the window value for 5 minutes at the 30-minute mark.

Through this, physiological mechanisms that regulate the activity of the glymphatic system may be discovered, and brainwave indices, near-infrared spectroscopy (NIRS) indices, and polysomnography indices that determine the activity of the glymphatic system may be discovered.

As shown in FIG. 6, the change in the average value of the brain water content may be measured between the first 5 minutes immediately after sedative administration and the last 5 minutes at the 30-minute mark. The change in average value of the brain water content according to sedative use and type is shown in Table 1 below.

**[Table 1]**

| **Change in average value of brain water content according to sedative use and type** | | | | |
|---|---|---|---|---|
| | Mean | Std. Error | 95% Confidence Interval | |
| | | | Lower Bound | Upper Bound |
| No sedation | 1.024 | .396 | .226 | 1.822 |
| Propofol | .503 | .276 | -.053 | 1.058 |
| Dexmedetomidine | 1.420 | .259 | .899 | 1.942 |

Furthermore, as shown in FIG. 7, it was confirmed that the brain water content increased at the 30-minute mark after sedative administration, i.e., administration of dexmedetomidine, compared to the baseline. The power spectrum analysis was performed by Fourier transforming the raw time series data of changes in brain water content to evaluate the power of very-low-frequency oscillation and low-frequency oscillation frequency bands (FIG. 8). In the above analysis, the very-low-frequency oscillation of the brain water content data is defined as 0.0067 to 0.04 Hz, and the low-frequency oscillation is defined as 0.04 to 0.1 Hz.

Alternatively, power spectrum analysis was performed by converting the raw time series data of brainwaves to evaluate the power for each frequency band (FIG. 9).

As shown in Table 1 below, when dexmedetomidine was administered, the brain water content increased to a greater extent than when propofol was administered to induce sedation. Although this difference was not statistically significant, it was observed that the group with sedatives showed a trend toward a greater increase in brain water content than the group without sedative administration.

**[Table 2]**

| **Changes in brain water content** | | | | | | |
|---|---|---|---|---|---|---|
| | | Mean Difference | Std. Error | Sig. | 95% Confidence Interval for Difference | |
| | | | | | Lower Bound | Upper Bound |
| No sedation | Propofol | .521 | .480 | .283 | -.446 | 1.489 |
| | Dexmedetomidine | -.396 | .477 | .411 | -1.357 | .565 |
| Propofol | No sedation | -.521 | .480 | .283 | -1.489 | .446 |
| | Dexmedetomidine | -.917 | .385 | .021 | -1.692 | -.143 |
| Dexmedetomi dine | No sedation | .396 | .477 | .411 | -.565 | 1.357 |
| | Propofol | .917* | .385 | .021 | .143 | 1.692 |

The brain water content during sedation with the sedative dexmedetomidine measured by NIRS increased and was significantly higher than that during sedation with the sedative propofol. This supports the conclusion that the activity of the glymphatic system may be activated by specific drugs. Furthermore, the potential utility of drugs, such as dexmedetomidine, for treating major diseases through activation of the function of the glymphatic system may be recognized. Additionally, this enables the discovery of biomarkers that evaluate whether and to what extent glymphatic activation mechanisms are operating.

As shown in Table 2, the increase in brain water content at the 30-minute mark compared to the baseline was associated with a decrease in absolute power in the alpha and beta bands of brainwaves.

**[Table 3]**

| **Main results of correlation analysis corrected for factors such as gender and age** | | | | | |
|---|---|---|---|---|---|
| | | Delta_abs_ratio | Theta_abs_ratio | Alpha_abs_ratio | Beta_abs_ratio |
| Delta (change in brain water content) | Correlation | .205 | .234 | -.453 | -.453 |
| | Significance (2-tailed) | .204 | .147 | .003 | .003 |
| | df | 38 | 38 | 38 | 38 |

Considering that the alpha and beta bands in brainwaves are frequency bands corresponding to brain activity in the arousal state, it was confirmed that the brain water content increased according to the level of decrease in arousal brainwaves caused by sedation, i.e., according to the presence or depth of sedation.

Table 3 illustrates correlation analysis to identify biomarkers associated with the increase in brain water content at the 30-minute mark relative to the baseline, after

correcting for factors such as gender and age. The increase in brain water content was confirmed to be associated with a high positive correlation with the very-low-frequency (VLF) oscillation component of the brain water-content signal.

**[Table 4]**

| **Discovery of biomarkers through association analysis corrected for factors such as gender and age** | | | | |
|---|---|---|---|---|
| | | AbsVLFl_ratio | AbsVLF1_delta | RelVLF1_delta |
| Delta (changes in brain water content) | Correlation | .086 | .219 | .495 |
| | Significance (2-tailed) | .599 | .174 | .001 |
| | df | 38 | 38 | 38 |

This demonstrates that the glymphatic activation mechanism, measured by the increased brain water content, may be regulated by very-low-frequency oscillation within the brain, and that the increase in very-low-frequency oscillation and/or low-frequency oscillation of the brain water content measured by NIRS may function as a biomarker for evaluating glymphatic activation and may be a key mechanism for inducing glymphatic activation. Therefore, very-low-frequency oscillation and/or low-frequency oscillation may be used as biomarkers for evaluating the operation and level of the glymphatic activation mechanism.

## Claims

1. A method for providing activity information or activity indices of the glymphatic system, performed by a processor, the method comprising:
analyzing changes in one or more brainwave indices and/or one or more NIRS indices associated with changes in brain water content of a subject's brain; and
providing activity or activity index information of the glymphatic system based on the analysis results.

2. The method of claim 1, further comprising:
analyzing very-low-frequency oscillation and/or low-frequency oscillation of the subject's brain;
providing brain water content information based on the analysis; and
providing activity information of the glymphatic system based on the brain water content information.

3. The method of claim 2, wherein the frequency of the very-low-frequency oscillation is from 0.0067 Hz to less than 0.04 Hz.

4. The method of claim 2, wherein the frequency of the low-frequency oscillation is from 0.04 Hz to 0.1 Hz.

5. The method of claim 2, wherein the level of increase in brain water content and glymphatic system activity is predicted based on the level of increase in relative power of the very-low-frequency oscillation and/or low-frequency oscillation.

6. The method of claim 1, wherein the analyzing of changes in the brainwave indices comprises analyzing brainwave spectrum power values.

7. The method of claim 6, wherein absolute power (uV²) of alpha [8 to 12 Hz] and/or beta [13 to 30 Hz] frequency bands is measured in the analyzing of the brainwave spectrum power.

8. The method of claim 7, wherein the level of increase in brain water content and glymphatic system activity is predicted by the level of decrease in absolute power in alpha and/or beta frequency bands.

9. The method of claim 7, further comprising analyzing connectivity within alpha [8 to 12 Hz] and beta [13 to 30 Hz] frequency bands of the brainwave spectrum.

10. The method of claim 9, wherein the analyzing of the connectivity within the frequency bands comprises measuring one or more indices selected from the group consisting of weighted phase lag index (wPLI), imaginary part of coherency (iCoh), and phase locking value (PLV).

11. The method of claim 1, wherein the subject is an individual administered with a sedative.

12. The method of claim 1, wherein the analyzing step comprises analyzing changes in polysomnography indices associated with changes in brain water content.

13. A computer-readable recording medium that stores program instructions operable by the computer, wherein when the program instructions are executed by a processor of the computer, the processor performs the method according to any one of claims 1 to 12.

14. An apparatus for measuring the activity of the glymphatic system, the apparatus comprising:
a first data processing unit that receives and analyzes information on one or more NIRS indices including very-low-frequency oscillation and/or low-frequency oscillation of a subject's brain while under a sedative, and brainwave indices including alpha [8 to 12 Hz] and/or beta [13 to 30 Hz] frequency bands of a brainwave spectrum; and
a second data processing unit that receives brain water content information for the subject and analysis results from the first data processing unit and provides information on the activity of the glymphatic system.

15. The apparatus of claim 14, wherein the sedative is dexmedetomidine.
